(19) Europäisches Patentamt
European Patent Office
Office européen des brevets

(11) Publication number: **0 119 737**
**A2**

(12) **EUROPEAN PATENT APPLICATION**

(21) Application number: **84300967.1**

(22) Date of filing: **15.02.84**

(51) Int. Cl.³: **A 61 K 47/00**, A 61 K 9/18

(30) Priority: **17.02.83 JP 25979/83**

(43) Date of publication of application: **26.09.84**
**Bulletin 84/39**

(84) Designated Contracting States: **BE CH DE FR GB IT LI NL SE**

(71) Applicant: **Takeda Chemical Industries, Ltd., 27, Doshomachi 2-chome Higashi-ku, Osaka-shi Osaka, 541 (JP)**

(72) Inventor: **Hirai, Shin-ichiro 201-202 Gouchi, *Tamamoto-cho Aburakojidori-shomensagaru,* Shimogyo-ku Kyoto 600 (JP)**
Inventor: **Jono, Kumiko, 36-203, 1 Terauchi 1-chome, Toyonaka Osaka 560 (JP)**
Inventor: **Shimizu, Hisayoshi, 6-20, Higashiohta 3-chome, Ibaraki Osaka 567 (JP)**

(74) Representative: **Laredo, Jack Joseph et al, Elkington and Fife High Holborn House 52/54 High Holborn, London, WC1V 6SH (GB)**

(54) **Pharmaceutical composition and its production.**

(57) An aqueous preparation, which comprises an active ingredient, cyclodextrin and a compound of the formula:

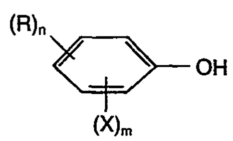

wherein R is alkyl, X is halogen, n is an integer of 0 to 2 and m is an integer of 1 to 3.
The use of the compound as the preservative in the present pharmaceutical does not result in decrease in antimicrobial activity.

EP 0 119 737 A2

# PHARMACEUTICAL COMPOSITION AND ITS PRODUCTION

This invention relates to a pharmaceutical composition which comprises an active ingredient, cyclodextrin and a preservative, and its production.

It is generally known that drugs which are strongly hydrophilic but have a small oil/water partition coefficient are poorly absorbable from the gastrointestinal tract and therefore are poor in bioavailability. Accordingly, such hydrophilic drugs are administered in the form of an injection so as to make their efficacy manifest to a satisfactory extent. However, administration by injection is entrusted only to experts and is accompanied by pain in patients. Therefore, a development of a preparation, other than an injection, which is capable of affording a high bioavailability and is applicable in an easy manner, has been desired. As a result of a study made for the purpose of increasing the bioavailability of such drugs being poorly absorbable from the gastrointestinal tract so as to make their pharmacological effects manifest in an efficient manner, it was found that addition of cyclodextrin to preparations other than an injection causes a significant increase in absorbability of the drugs and that addition of cyclodextrin to preparations containing other drugs than those mentioned above also results in improvement in the stability and solubility of active ingredients. Then, the present inventors prepared aqueous preparations containing active ingredients and cyclodextrin and added preservatives

thereto. Regrettably, however, a phenomenon that the preserving effect of the preservative added is decreased was observed in some of such aqueous preparations.

Under these circumstances, the present inventors conducted an intensive study to find out an aqueous preparation which contains an active ingredient, cyclodextrin and a preservative but does not show a decrease in the preservative effect and found that this object can be achieved by the use of phenol derivatives as preservatives. Continued research based on this finding has now led to completion of the present invention.

The invention thus provides an aqueous preparation which comprises an active ingredient, cyclodextrin and a compound of the formula:

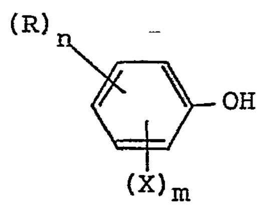

(I)

wherein R is alkyl, X is halogen, n is an integer of 0 to 2 and m is an integer of 1 to 3.

The alkyl represented by R in the compound (I) to be contained in the aqueous preparation according to the invention preferably contains 1 to 8 carbon atoms. Examples are methyl, ethyl, n-propyl, n-butyl, n-amyl, n-hexyl, n-heptyl and n-octyl, among others.

The halogen represented by X is, for example, chlorine or bromine.

Examples of the compound (I) to be used in accordance with the invention are o-chlorophenol, m-chlorophenol, p-chlorophenol, o-bromophenol, m-bromophenol, p-bromophenol, 2,4-dichlorophenol, 2,4,6-trichlorophenol, 2,4,6-tribromophenol, p-chloro-m-cresol, p-bromo-m-cresol, 2-n-butyl-p-chlorophenol, 2-n-amyl-p-bromophenol, p-chloro-m-xylenol, p-bromo-m-xylenol, 4-ethyl-o-chlorophenol, 2-ethyl-p-chlorophenol, 2-n-propyl-p-chlorophenol, amongst others.

The active ingredient in the aqueous preparation according to the present invention is not limited to any specific class. Thus, said active ingredient includes, among others, physiologically active polypeptide drugs, polysaccharide drugs, aminoglycoside drugs, beta-lactam antibiotics, nucleic acid drugs and other hydrophilic drugs and various lipophilic drugs.

The polypeptides are exemplified by peptides which comprises two or more than two amino acid residues. The polypeptides preferably have a molecular weight of from 200 to 60000.

As the physiologically active polypeptide, there may be mentioned, for example, L-pyroglutamyl-L-histidyl-L-prolinamide (thyrotropin releasing hormone; hereinafter referred to briefly as TRH) or its salts, especially its tartrate [U.S. Patent No. 3,957,247], and polypeptide represented by the formula (II)

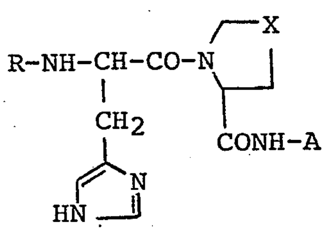

(II)

wherein A stands for hydrogen, alkyl, aralkyl, alkoxyalkyl, hydroxyalkyl or alkoxy, R stands for 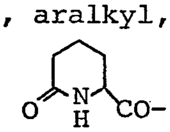 , 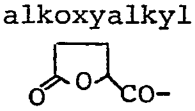 or 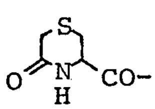 , X stands for $-CH_2-$, $-CH_2CH_2-$ or -S-, R and each of the other constituent amino acid residues may have an L- or D-configuration or be racemic] and salts thereof [U.S. Patent No. 4,100,152].

Among the compounds represented by the formula (II), the compound shown below is referred to briefly as "DN-1417".

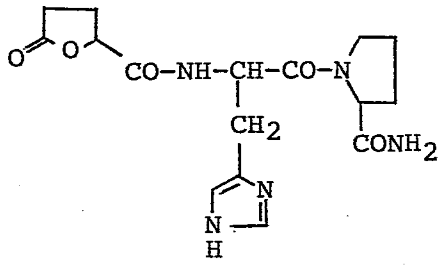

· citrate

(γ-butyrolactone-γ-carbonyl-L-histydyl-L-prolinamide· citrate)

Furthermore, as the polypeptide, there may be mentioned luteinizing hormone-releasing hormone (hereinafter referred to briefly as "LH-RH") or peptides which have the LH-RH activity and have the formula (III).

(Pyr)Glu-$R_1$-Trp-Ser-$R_2$-$R_3$-$R_4$-Arg-Pro-$R_5$ (III)

[wherein $R_1$ stands for His, Tyr, Trp or p-$NH_2$-Phe; $R_2$ stands for Tyr or Phe; $R_3$ stands for Gly or a D-amino acid residue: $R_4$ stands for Leu, Ile or Nle; $R_5$ stands for Gly-NH-$R_6$ ($R_6$ stands for H or a lower alkyl group which may optionally have a hydroxyl group) or NH-$R_6$ ($R_6$ has the meaning defined above)] [U.S. Patent No. 3,853,837, U.S. Patent No. 4,008,209, U.S. Patent No. 3,972,859. British Patent No. 1,423,083, Proceedings of the National Academy of Science of the United States of America, vol. 78, pp. 6509-6512 (1981)].

As examples of the D-amino acid residue $R_3$, there may be mentioned the residues of alpha-D-amino acids containing up to 9 carbon atoms (e.g. D-Leu, Ile, Nle, Val, Nval, Abu, Phe, Phg, Ser, Thr, Met, Ala, Trp, α-Aibu, etc.), which may have suitable protective groups (e.g. t-butyl, t-butoxy, t-butoxycarbonyl, etc.). Of course, salts of peptide (III) with acids as well as metal complex compounds of peptide (III) may also be employed just as peptide (III). All abbreviations, wherever they are used in this specification to denote amino acids, peptides, protective groups, etc., are those according to IUPAC-IUB Commission on Biochemical Nomenclature or those commonly employed in the

particular field of art. Where any of the amino acids named herein is subject to optical isomerism, all references to such amino acid mean the L-form unless otherwise indicated.

In the present specification, the polypeptide (III) in which $R_1$=His, $R_2$=Tyr, $R_3$=D-Leu, $R_4$=Leu, $R_5=NHCH_2-CH_3$ is referred to briefly as TAP-144.

Examples of said polypeptide include insulin, somatostatin, somatostatin derivatives (U.S. Patent No. 4,093,573, U.S. Patent No. 4,100,117, U.S. Patent No. 4,253,998), growth hormone, prolactin, adrenocorticotrophic hormone (ACTH), melanocyte stimulating hormone (MSH), thyroid stimulating hormone (TSH), luteinizing hormone (LH), follicle stimulating hormone (FSH), vasopressin, vasopressin derivatives {desmopressin [Folia Endocrinologica Japonica, 54, 5, pp. 676-691, (1978)]}, oxytocin, carcitonin, parathyroid hormone, glucagon, gastrin, secretin, pancreozymin, cholecystokinin, angiotensin, human placental lactogen, human chorionic gonadotropin (HCG), enkephalin, enkephalin derivatives [U.S. Patent 4,277,394; European Patent Application Publication No. 31567], endorphin, interferon (α, β, γ), interleukin (1, 2), urokinase, kallikrein, thymopoietin, thymosin, motilin, dynorphin, bombesin, neurotensin, caerulein, bradykinin, substance P, kyotorophin, fertirelin acetate, nerve growth factor, peptide type antibiotics such as polymyxin B, colistin, gramicidin, bacitracin, peptide type anti-tumor agents such as bleomycin, neocarzinostatin.

The polysaccharide drugs mentioned above include, among others, heparin and such antitumor agents as lentinan, zymosan and PS-K (krestin).

The aminoglycoside antibiotics mentioned above include, among others, gentamycin, streptomycin, kanamycin,

dibekacin, paromomycin, kanendomycin, lipidomycin, tobramycin, amikacin, fradiomycin and sisomicin.

The beta-lactam antibiotics mentioned above include, among others, penicillins such as sulbenicillin, mecillinam, carbenicillin, piperacillin and ticarcillin, thienamycin, and cephalosporins such as cefotiam, cefsulodine, cefmenoxime, cefmetazole, cefazolin, cefotaxime, cefoperazone, ceftizoxime and moxalactam.

The nucleic acid drugs mentioned above include, among others, citicoline and such antitumor agents as cytarabine and 5-FU (5-fluorouracil), 1-(2-tetrahydrofuryl)-5-fluorouracil.

The other hydrophilic drugs are exemplified by doxorubicin, mitomycin C, glutathion, gamma globulin and lysozyme.

The lipophilic drugs are exemplified by nalidixic acid, cephalexin, cephalothin, cephamandole, ampicillin, amoxicillin, talampicillin, cyclacillin, minocycline, futrafur, phenytoin, hexylresorcinol, aspirin, tolbutamide, clofibrate, pentazocine, nitrofurantoin, clonidine, tocopherol, diclofenac, dexamethazone, nifedipine, lincomycin, rifampicin, ibuprofen, mecobalamin, spironolactone, indometacin, diazepam, cimetidine, ranitidine, propranolol, methyldopa, sulindac, chlorpramide, paracetamol, allopurinol, flurazepam, isosorbide, nitroglycerin and so on.

The cyclodextrin to be used in the aqueous preparation according to the invention includes various cyclodextrin species obtained by hydrolysis of starch with an acid or amylase and, in addition, cyclodextrin derivatives.

Said cyclodextrin species include α-cyclodextrin (polymerization degree: 6), β-cyclodextrin (polymerization degree: 7) and γ-cyclodextrin (polymerization degree: 8) [cf. Biochemical and Biophysical Research Communications vol. 5, pages 11-15 (1961), Farumashia, Vol. 16, No. 1, pages 33-37 (1980); Yakugaku Zasshi (Journal of the Pharmaceutical Society of Japan), Vol. 101, No. 10, pages

857-873 (1981), Japanese Patent Application Publication Sho. 53(1978)-31,223].

Said cyclodextrin derivatives include, among others, tri-O-methylcyclodextrin [cf. Chemical & Pharmaceutical Bulletin, Vol. 28, pages 1552-1558 (1980)] and triamino-cyclodextrin [cf. Angewandte Chemie, International Edition in English, Vol. 19, pages 344-362 (1980)].

The aqueous preparation according to the invention may take the form of liquid preparations for injection, liquid preparation for internal use (e.g. extracts, elixirs, syrups, infusions, decoctions, suspensions, emulsions, aromatic waters, lemonades, fluid extracts), liquid preparations for external use (e.g. eye drops, aqueous preparations for rectal administration, aqueous preparations for vaginal administration, aqueous ointments, nasal drops, ear drops, cataplasms, liniments, lotions), etc.

The aqueous preparation according to the invention contains water in an amount of from 10 to 99.9 percent (weight by volume), preferably from 20 to 99 percent (weight by volume).

The aqueous preparation can be produced by any of the *per se* known methods. Thus, for instance, an injection can be produced by dissolving an effective amount of the active ingredient and cyclodextrin in distilled water for injection, further adding an isotonizing agent, a buffer and the preservative according to the invention and, after complete dissolution, filtering the solution through a Millipore filter (Millipore Corporation, USA), followed by filling the filtrate into vials, sealing the vials and high-pressure steam sterilization at 115.5°C for 30 minutes. The injection may contain a stabilizer, a solubilizing agent and/or a local anesthetic, for instance, in addition to the above-mentioned components.

A liquid preparation, for instance a syrup, can be produced by adding an effective amount of the active ingredient, cyclodextrin, a thickening agent (e.g. sodium

carboxymethyl cellulose, methyl cellulose), sucrose, a flavoring agent, etc. to a simple syrup or water with the presevative according to the invention dissolved therein in advance, followed by stirring for homogenization and adjusting the volume with water.

A liquid preparation for external use, for example a nasal drop preparation, can be produced by adding an effective amount of the active ingredient, cyclodextrin, an isotonizing agent, a buffer, a flavoring agent, etc. to an aqueous solution of the preservative according to the invention and, after complete dissolution, filtering the solution and filling the filtrate into containers for nasal application.

The concentration of cyclodextrin in the preparation is generally 0.1 to 50 percent (weight by volume), preferably 0.5 to 20 percent (weight by volume), more preferably 1 to 10 percent (weight by volume).

The phenol derivative (I) is used in the aqueous preparation according to the invention in a concentration not lower than the minimal concentration required to inhibit the growth of microorganisms (MIC, minimal inhibitory concentration), preferably in a concentration of from 0.001 to 1 percent (weight by volume), more preferably from 0.01 to 1 percent (weight by volume).

A characteristic feature of the invention lies in that the preservative is added for the purpose of guaranteeing, for a long period of time, the quality of aqueous preparations with cyclodextrin being incorporated therein so as to stabilize the active ingredient, improve the solubility and increase the bioavailability, and for other purposes. The use of the preservative according to the invention does not result in decreases in antimicrobial activity due to interaction with cyclodextrin and does not result in precipitation of an insoluble complex; hence, the use of the present preservatives can give effective aqueous preparations.

The following Experimental Examples and Examples illustrate the invention in more detail. In the following description, "percent (%)" means "weight/volume percent" unless otherwise specified.

Experimental Example 1

Various preservative solutions are tested for the minimal inhibitory concentration (MIC) using Aspergillus niger ATCC 9642 (A. niger) and Escherichia coli IFO 3044 (E. coli) as the test organisms. Thus, two culture media are prepared by adding 0% and 5% of α-cyclodextrin, respectively, to a 1/15 M phosphate buffer (pH 6.5) containing 2% of Polypeptone (Daigo Nutritive Chemicals Ltd., Japan). The preservative is added to each medium and the resulting solution is diluted serially with each of the respective media to give solutions differing in preservative concentration. The test organisms are added to these solutions to a final concentration of $10^5$ cfu/ml (cfu: colony forming unit). After incubation at 25°C (when A. niger ATCC 9642 is used) for 5 days or 35°C (when E. coli IFO 3044 is used) for 3 days, the minimal inhibitory concentration is measured by observation with the naked eye. The results obtained in this manner are shown in Table 1.

Table 1 Minimal inhibitory concentration (MIC, μg/ml)

| Preservative | Culture medium without addition of cyclodextrin | | Culture medium + 5% α-cyclodextrin | |
|---|---|---|---|---|
| | A. niger ATCC 9642 | E. coli IFO 3044 | A. niger ATCC 9642 | E. coli IFO 3044 |
| [Controls] | | | | |
| Methylparaben | 500 μg/ml | 500 μg/ml | 4000 μg/ml | 4000 μg/ml |
| Ethylparaben | 250 | 500 | 4000 | 4000 |
| Propylparaben | 125 | 250 | 4000 | 4000 |
| Butylparaben | 125 | 125 | 4000 | 4000 |
| [This Invention] | | | | |
| p-Chlorophenol | 200 | 200 | 400 | 400 |
| p-Chloro-m-cresol | 100 | 200 | 200 | 200 |
| p-Chloro-m-xylenol | 50 | 100 | 75 | 150 |

As is evident from Table 1, the parabens used as controls (methylparaben, ethylparaben, propylparaben and butylparaben) each show a significant increase in the value of minimal inhibitory concentration in the cyclodextrin-containing culture medium, hence a significant decrease in the antimicrobial activity. On the other hand, the preservatives according to this invention each exhibit only a slight decrease in the antimicrobial activity even in the cyclodextrin-containing culture medium.

Experimental Example 2

An aqueous solution containing 5% of α-cyclodextrin or 1% of β-cyclodextrin was prepared and each of various preservatives was dissolved therein. After standing in a cold place overnight, the solution was examined for the presence or absence of a precipitate. The results obtained are shown in Table 2.

Table 2

| Preservative | Concentration (%) | 5% α-Cyclodextrin | 1% β-Cyclo dextrin |
|---|---|---|---|
| [Controls] | | | |
| Methylparaben | 0.5 | ++ | ++ |
| Propylparaben | 0.1 | + | + |
| Benzalkonium chloride | 0.03 | + | + |
| Benzethonium chloride | 0.03 | + | + |
| Chlorobutanol | 0.3 | ++ | ++ |
| Chlorhexidine gluconate | 0.1 | ++ | ++ |
| Benzyl alcohol | 0.5 | + | + |
| Phenethyl alcohol | 0.1 | + | + |
| Cetylpyridinium chloride | 0.03 | + | + |
| [This invention] | | | |
| p-Chlorophenol | 0.05 | - | - |
| p-Chloro-m-cresol | 0.02 | - | - |
| p-Chloro-m-xylenol | 0.02 | - | - |

Notes: -, no precipitation; + precipitation; ++, precipitation in a large amount.

As is evident from Table 2, a precipitate forms in cyclodextrin-containing solutions with each of the preservatives used as controls. On the other hand, the preservatives according to the invention give no precipitate. Therefore, it is considered that the interaction between the preservatives used in this invention and cyclodextrin is slight and accordingly does not result in a decrease in the antimicrobial activity.

Example 1

A preparation for nasal application by dropping is produced by dissolving 5 g of α-cyclodextrin and 1.8 g of glycerol as an isotonizing agent in 80 ml of purified water, heating the solution to 80°C, adding 30 mg of p-chloro-m-xylenol as a preservative and 10 mg of l-menthol as a flavor at that temperature, cooling, after complete dissolution, to 25°C, adding 5 g of leuprolide [TAP-144: (Pyr)Glu-His-Trp-Ser-Tyr-D-Leu-Leu-Arg-Pro-$NHCH_2$-$CH_3$ (abbreviations for amino acids being by IUPAC-IUB Commission on Biochemical Nomenclature)], and, after dissolution, further adding purified water to make 100 ml.

Example 2

A preparation for nasal application by dropping is produced by completely dissolving 5 g of α-cyclodextrin, 5 g of protirelin [thyrotropin releasing hormone (TRH): L-pyroglutamyl-L-histidyl-L-prolinamide]·tartrate, 600 mg of sodium chloride and 20 mg of p-chloro-m-xylenol as a preservative in 100 ml of purified water.

Example 3

A preparation for nasal application by dropping is produced by completely dissolving 100 mg of fertirelin acetate, 1 g of β-cyclodextrin, 800 mg of sodium chloride and 50 mg of p-chloro-m-xylenol as a preservative in 100 ml of purified water.

Example 4

A hydrogel eyedrop is produced by adding 634 mg of β-cyclodextrin and further 50 mg of 2,4-dichlorophenol as a preservative to 200 mg of indomethacin and 117 mg of L-arginine, adding distilled water to make the whole amount 100 ml, adding 2 g of methylcellulose (4,000 cp) thereto and stirring the mixture under ice cooling.

Example 5

An injection is produced by dissolving 10 mg of prostaglandin, 150 mg of α-cyclodextrin and 100 mg of p-bromophenol as a preservative in 100 ml of distilled water for injection and filling the solution into vials, followed by high-pressure steam sterilization at 115.5°C for 30 minutes.

Example 6

A suspension for internal application is produced by adding 100 g of β-cyclodextrin, 500 mg of 2-n-amyl-p-bromophenol as a preservative and 500 ml of purified water to 10 g of 1-(2-tetrahydrofuryl)-5-fluorouracil, followed by mixing.

Example 7

A syrup for internal application is produced by adding 20 ml of water to 10 g of nalidixic acid and 5 g of β-cyclodextrin, mixing well, adding 0.8 g of sodium carboxymethylcellulose, 2 ml of ethanol and 60 ml of simple syrup, further dissolving 50 mg of p-chloro-m-xylenol as a preservative and making the whole volume 100 ml.

Example 8

An injection is produced by dissolving 5 x $10^8$ units of γ-interferon, 100 mg of β-cyclodextrin, 80 mg of sodium chloride and 50 mg of p-chloro-m-xylenol as a preservative

in 10 ml of distilled water for injection and, after sterile filtration, filling the solution into 1-ml vials.

Example 9

An injection is produced by dissolving $10^4$ units of interleukin 2, 500 mg of α-cyclodextrin, 80 mg of sodium chloride and 4 mg of p-chloro-m-cresol as a preservative in 10 ml of distilled water and, after sterile filtration, filling the solution into 1-ml vials.

Example 10

A suspension for external application is produced by adding 50 g of β-cyclodextrin, 100 mg of p-bromo-m-cresol as a preservative and 500 ml of purified water to 2 g of isosorbide, followed by mixing.

Example 11

A syrup for internal application is produced by adding 20 ml of water to 5 g of cimetidine and 10 g of α-cyclodextrin, adding 1.2 g of methylcellose, 60 ml of simple syrup, further dissolving 100 mg of p-bromo-m-xylenol as a preservative and making the whole volume 100 ml.

CLAIMS:

1. An aqueous preparation which comprises an active ingredient, cyclodextrin and a compound of the formula:

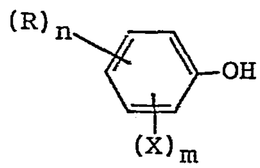

wherein R is alkyl, X is halogen, n is an integer of 0 to 2 and m is an integer of 1 to 3.

2. A preparation as claimed in Claim 1, wherein X is chlorine.

3. A preparation as claimed in Claim 1, wherein X is bromine.

4. A preparation as claimed in Claim 2, wherein the compound is p-chloro-m-xylenol or p-chloro-m-cresol.

5. A preparation as claimed in Claim 3, wherein the compound is p-bromo-m-cresol or p-bromo-m-xylenol.

6. A preparation as claimed in Claim 1, wherein the active ingredient is a physiologically active polypeptide drug.

7. A preparation as claimed in Claim 6 wherein the physiologically active polypeptide drug is (Pyr)Glu-His-Trp-Ser-Tyr-D-Leu-Leu-Arg-Pro-$NHCH_2CH_3$.

8. A preparation as claimed in Claim 6, wherein the physiologically active polypeptide drug is L-pyroglutamyl-L-histidyl-L-prolinamide·tartrate.

9. A preparation as claimed in Claim 1, wherein the cyclodextrin is α-cyclodextrin.

10. A preparation as claimed in Claim 1, wherein the cyclodextrin is β-cyclodextrin.